# EUROPEAN PATENT SPECIFICATION

(11) **EP 1 085 020 B1**
(45) Date of publication and mention of the grant of the patent: **17.03.2004**
(21) Application number: 00203172.2
(22) Date of filing: 12.09.2000
(51) Int. Cl.: C07D 473/16, C07D 487/04

(54) **Synthesis of peptide nucleic acids**
Synthese von Peptid-Nucleinsäuren
Synthèse d'acides nucléiques peptidiques

(30) Priority: 17.09.1999 DK 132299
(43) Date of publication of application: 21.03.2001
(73) Proprietor: PNA Diagnostics ApS Applied Biosystems, 2850 Naerum (DK)
(72) Inventor: Batz, Hans-Georg Dr., 82327 Tutzing (DE); Hansen, Henrik Frydenlund, 2660 Roedovre (DK); Oerum, Henrik Dr., 3500 Vaerloese (DK); Koch, Troels Dr., 2300 Copenhagen S (DK); Kofoed, Thomas, 5260 Odense S (DK)
(74) Representative: Christiansen, Ejvind

(56) References cited:
- EP-A- 0 212 535
- WO-A-00/43394
- WO-A-98/16528
- WO-A-99/07705

## Description

Subject of the present invention is a method for the preparation of peptide nucleic acids.

Non-natural nucleic acid binding compounds have been found to be valuable mimics for nucleic acids, especially mimicking or even improving the nucleic acid binding features of DNA and RNA. Peptide Nucleic Acids (PNA) have been shown to form thermally very stable duplexes and triplexes with complementary RNA and DNA oligonucleotides (Egholm et al. Nature, 1993, 365, 566-568). Further substitution of adenine (A) by 2,6-diaminopurine (D) has been reported to raise the melting point (Tₘ) for DNA/DNA and RNA/DNA hybrids (Hoheisel et al. FEBS 1990, 274, 103; Lamm et al. Nucleic Acids Research 1991, 19, 12 3193). This effect on the Tₘ is depending upon the nucleo base sequence of the DNA oligonucleotide. Thus, the stabilization compared to duplexes formed from the DNA and an oligonucleotide containing only A is most pronounced in mixed sequences. There may even be a destabilizating effect in poly adenine sequences (Cheong et al., Nucleic Acids Research, 1988, 16,11, 5115).

Subject of the present invention is a method for the synthesis of compounds of the general formula I 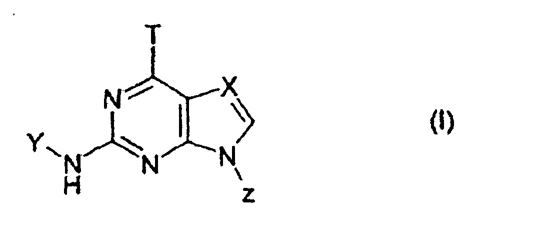 wherein
T is NV¹V²
wherein V¹ and V² are linked together other than via the N shown in the definition by a chain of at least 5 atoms, or T is NHV, wherein V is selected from the group consisting of hydrogen, alkyl and a protecting group,
X is CH or N,
Y is an electron withdrawing protecting group, preferably a group belonging to the group of acyl groups, unsubstituted or substituted, and
Z is an alkyl group substituted with a derivative of a COOH group wherein the derivative is an ester, thioester or amide with an unprotected or protected peptide nucleic acid,
characterized in that a compound of general formula II 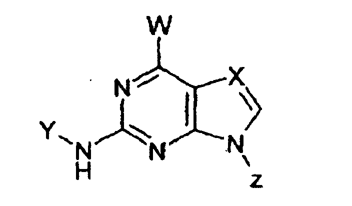 wherein
the definitions of X, Y and Z are chosen from the possible definitions of X, Y and Z in formula I and
W is an electron withdrawing group, preferably selected from the group consisting of NO₂, halogen, Tos and Mes,
is reacted with a compound of general formula III,

T-H (III)

wherein T is defined as in formula I,
under non-alkaline conditions favoring the elimination of a compound of the formula WH.

Any substituent in Z may or may not be altered during the reaction to yield another substituent within the possible definitions of said substituents in Z.

Tos means the tosyl group and Mes means the mesyl group. Preferred halogens are iodine (I), Br and Cl, most preferred Cl.

Preferred definition of T comprising linked substituents V¹ and V² are N-heterocyclic groups derived from secondary amines, like the piperidyl group or the morpholino group. However, such definition will also include compounds comprising aromatic moieties or other double bond containing moieties, as long as at least one secondary amino group is contained. The chain of atoms will be preferably from 5 to 8, most preferably 6, atoms long. Preferred atoms in the chain are carbon atoms and nitrogen and oxygen atoms. A preferred definition of V is the benzyloxycarbonyl group. Preferred amino protecting groups V are groups that can be selectively removed after the synthesis from the exocyclic amino group.

Alkyl groups in the definitions of all formulae, if not otherwise mentioned, preferably contain from 1 to 6, most preferably from 1 to 3, carbon atoms. Acyl groups preferably contain from 2 to 21 carbon atoms, including acyl groups from alkanes including from 1 to 6 carbon atoms unsubstituted or substituted by one or more aromatic groups of from 6 to 14 carbon atoms, and from aromatic moieties, like arenes including from 6 to 14 carbon atoms. Preferred alkylacyl groups are isobuturyl, t-buturyl, isopropy and acetyl and preferred aromatic acyl is benzoyl. An aromatic group or aryl group is a group containing at least one aromatic ring, unsubstituted or substituted by one or more, but usually less than 3, substituents selected from the group consisting of hydroxyl, alkoxy, alkyl, nitro, halogen or carboxyl. Preferred aromatic groups are the phenyl and the natpthyl group.

A protecting group of a particular group is a group which prohibits the particular group to participate in any undesired chemical reactions. Preferably, the protecting group can be removed from the particular group thus setting the particular group free. Protecting groups, for example for amino groups, hydroxyl groups and carboxyl groups are generally wellknown to the man skilled in the art, for example from text books on oligonucleotide or peptide synthesis. Protecting groups for amino groups include acyl groups or carbonyloxyalkyl, for hydroxyl groups include acyl groups and for carboxyl groups include alkyl groups. The electron withdrawing and electron donating capability is compared to hydrogen.

An aralkyl group is an alkyl group substituted by an aryl group. Preferred aralkyl is the benzyl group.

Preferred definitions of T consist of NH₂, NHCH₃ and piperidyl.

The preferred definition of X is N.

The preferred definition for Y is iso-butyryl (C₃H₇CO).

All these compounds of general formula I can according to the present invention be prepared by substituting group W by the amino group of the compound of formula III. This reaction can be considered as a nucleophilic substitution reaction driven by the exit of a compound WH. Aromatic nucleophilic substitutions at heterocycles are principally known to a man skilled in the art, but have never been applied to diaminopurines.

Nucleic acid binding compounds are defined according to the present invention by their capability of binding to a nucleic acid via hydrogen bonds of basepairing between complementary bases of both the nucleic acid and the nucleic acid binding compound. The binding can be either in duplex form (for example containing one strand of a nucleic acid and one strand of the nucleic acid binding compound) or in triplex form (for example containing one strand of the nucleic acid and two strands of the nucleic acid binding compound). Preferred nucleic acid binding compounds are not naturally occurring. Examples are disclosed in

Jones, A.S., Int. J. Biol. Macromol. 1979, I, 194; De Koning, H., Pandit, U.K., Rec. Trav. Chim., 1971, 91, 1069; Takemoto, K, Inaki, Y., Polym. Mat. Sci. Eng., 1988, 58, 250; Weller, D.D., Daly, D.T., Olsen, W.K., Summerton, J.E., J. Org. Chem., 1991, 56, 6000; Garner, P., Yoo, J.U., Tett. Lett., 1993, 34, 1275; EP-A-0 672 700; EP 0 646 595 or WO 86/05518.

Most preferred nucleic acid binding compounds are disclosed in WO 92/20702. To this most preferred group of nucleic acid binding compounds further any derivatives and specific embodiments are counted. In WO 94/25477 novel peptide nucleic acids are described, WO 96/02558 is directed to linked PNAs and WO 96/20212 is directed to PNAs incorporating a chiral backbone. WO 95/14708 is directed to compounds having chelating moieties bound to the nucleic acid binding compounds and WO 95/16202 is directed to nucleic acid binding compounds having an enzyme substrate attached thereto, WO 95/08556 discloses chimeric molecules of nucleic acids and nucleic acid binding compounds. The synthesis of such compounds is described in detail therein. Therefore, reference is made with respect to the chemical structure especially the backbone, to these documents. These nucleic acid binding compounds are usually prepared using a strategy requiring protection of any functional groups within the compound during extension of the backbone, such that any functional groups like primary amino groups or hydroxy groups not intended to be extended in the backbone extension are protected by appropriate protecting groups which can be removed in a final step to set free the functional groups in the nucleic acid binding compound. Such functional groups include preferentially any amino groups at nucleobase moieties, like the exocyclic amino groups of cytosine, adenine and guanine. Such protecting groups are known from WO 92/20702, WO 93/12129 and WO 95/17403.The most preferred protecting group hitherto known is the acyl group and the Z group (benzyloxycarbonyl). For any amino groups at the backbone, the Boc group (tert-butyloxycarbonyl) is well established.

Within peptide nucleic acid, such groups include a carboxyl group and an amine group, preferentially primary amine group. As disclosed in WO 92/20702, the amine group is preferentially protected by the Boc group, but other protecting groups may be the Mmt or the Fmoc groups. Carboxyl groups may be protected using the benzyl group (Bn) forming a carboxylic acid ester. The term "partially protected" means that not all functional groups are protected, but at least one of them. While the fully protected monomeric backbone units may be produced preferentially in the synthesis of such monomeric backbone unit partially protected backbone units are the preferred starting compounds for attaching the monomeric backbone unit to solid phase, a further monomeric backbone unit or a nucleic acid binding compound to be extended. Preferentially, in case of peptide nucleic acid, such a partially protected monomeric backbone unit contains protected primary amino group as well as a free carboxylic acid group.

Also described herein is a method for the preparation of compounds of formula I, wherein Z is a monomeric backbone unit of a nucleic acid binding compound comprising reacting a compound of formula I, wherein Z is R', wherein R' is -(CR¹R²)ₙ-CO-OR³, R¹ and R² for each C at different n are chosen independently from the group consisting of hydrogen, alkyl, aryl or allyl or R¹ and R² at one C at a defined position are together oxygen and R³ is an alkyl or aralkyl group and n is an integer of from 1 to 5 with a monomeric backbone unit containing a not protected amino group under conditions favoring the elimination of an alkyl or aralkyl alcohol.

Surprisingly, such monomers can be synthesised in high yield and with reduced loss of protecting groups by reacting a compound of formula II, wherein Z is a partially or fully protected monomeric backbone unit of a nucleic acid binding compound with a compound of formula III as outlined above. However, in order to avoid the preparation of guanidine side products, alkaline conditions should be avoided. The useful synthesis has been proven for ammonia and other primary amines and it is expected, based on these experiments, that it will work also for other nucleophilic compounds.

In an overall view of monomer synthesis, the synthesis of monomers via compounds of formula II has the principal advantage that the route of synthesis for diaminopurine derivatives and guanine derivatives is the same for a large number of steps. In order to prepare guanine derivatives instead of diaminopurine derivatives, a compound of formula II which could be used for diaminopurine synthesis, can be subjected to alkaline condition, thus replacing the substituent N in formula II by hydroxyl.

As outlined above, diaminopurine containing nucleic acid binding compounds have particular properties, especially in PNAs. The controlled chemical synthesis of nucleic acid binding compounds usually comprises oligomerisation of monomeric backbone units which are suitably protected for avoiding side reactions, both of the monomer and of the already prepared oligomer, only letting free the functional groups for the intended coupling reaction. This new synthesis step can be used to prepare protected aminopurine moieties. It must be understood that protected nucleobases or their analogues, as aminopurine, are advantageously used during the synthesis of nucleic acid binding compounds because the extension of such nucleic acid binding compounds containing such protected diaminopurine moieties goes on without less side-reactions created by the amino groups if they were not protected. However, the prior art did not provide for easy synthesis of diprotected diaminopurine moieties.

The present invention, in contrast to the prior art, in a preferred embodiment will comprise the chemical synthesis of a precursor oligomer using at least one monomeric compound of formula II, wherein Z is a partially protected monomeric backbone unit. Such synthesis can be performed analogously to the method as for the monomers containing other (naturally occurring) nucleobases, avoiding alkaline conditions. The overall synthesis will yield an oligomer which is partially or totally protected, comprising at least one heterocycle containing W. Thereafter, group W is replaced by T as outlined above, using nucleophilic replacement conditions.

If a nucleic acid binding compound contains more than one group wherein this replacement reaction is intended to occur, this may be done in one step. Further, under the conditions of this replacement reaction there may be further alterations within the nucleic acid binding compound or the monomeric backbone unit, such as simultaneous removal of protecting groups. Surprisingly, this reaction can be conducted with high yield and again with a number of nucleophilic compounds of formula III. After oligomerisation and replacement, the partially or totally protected compound can be treated further, for example, any protecting group including those present in T, can be removed unselectively or selectively to attach reporter groups (like fluorescein), solid phases (like carboxyl group containing cellulose) or just to produce the binding properties of the oligomer.

Most preferred nucleic acid binding compounds of formula I comprise at least one monomeric subunit of the general formula IX: 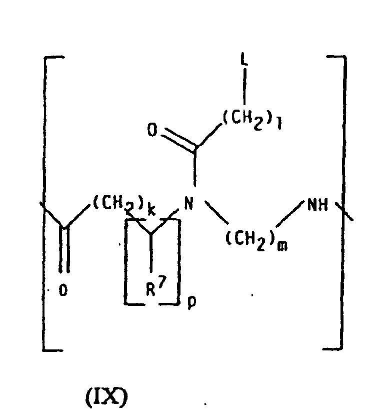 wherein
L is a protected diaminopurine as pointed out above,
k, I and m is independently zero or an integer from i to 5,
p is zero or 1, and
R⁷ is selected from the group consisting of hydrogen and the side chains of naturally occurring alpha amino acids.

Figure 5 shows the synthesis of a protected diaminopurine containing PNA monomers 7, 8, 9, 11 and 12 as well as the (heterocyclus) unprotected monomer 10.

Figure 6 shows the nucleophilic displacement in a PNA oligomer, using as Nucleophile (:Nu) either ammonia or methyl amine.

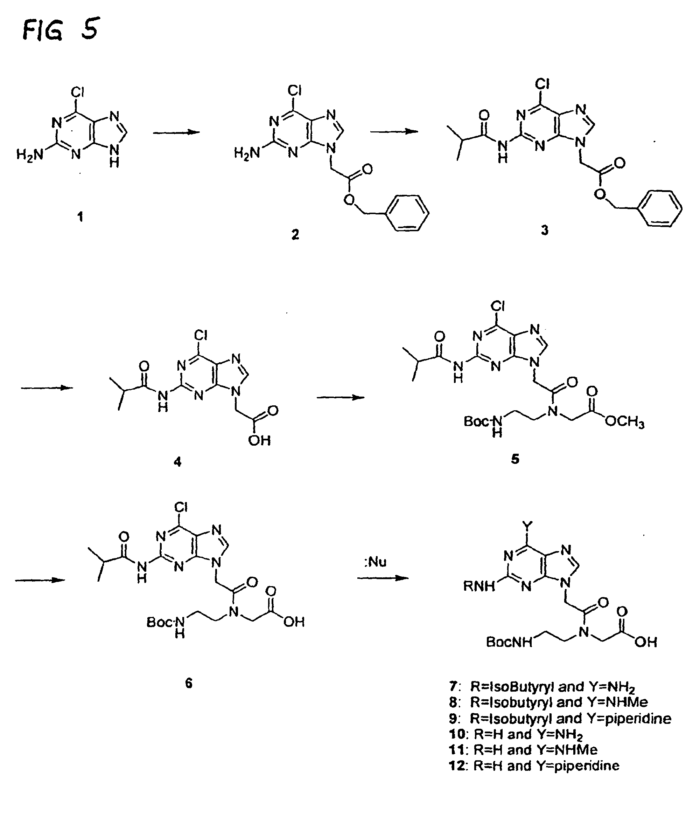

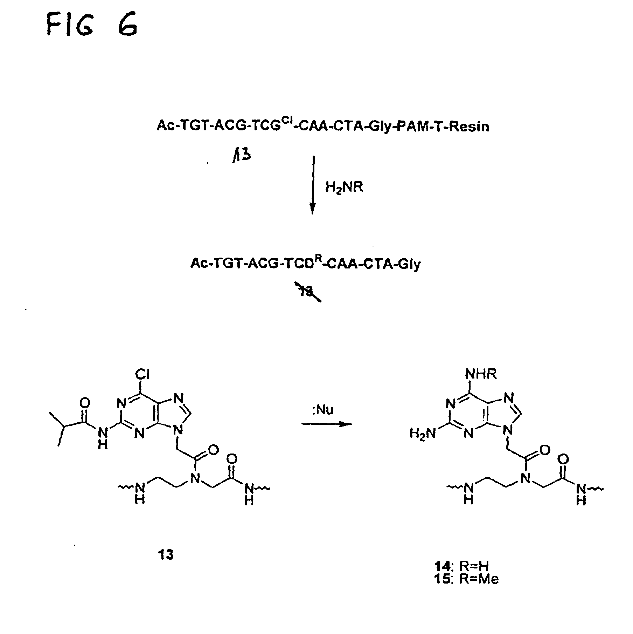

The following examples are explaining the invention in more detail.

### Examples

### General

All reagents used are analytical grade and from Aldrich. HATU was purchased from PerSeptive Biosystems (MA). MBHA resin were purchased from NovaBiochem (Switzerland).

### Abbreviations

The following abbreviations are employed: triethylamine (TEA), *tert*-butyloxycarbonyl (Boc), dimethylformamide (DMF), dichloromethane (DCM), butyl (Bu), tetrahydrofurane (THF), dimethylsulfoxide (DMSO) and acetyl (Ac).

### Example 1

### Benzyl (2-amino-6-chloropurin-9-yl)acetate (2) Synthesis of Boc 6-Chloro-N²-Isobutyryl Guanine Monomer (Figure 5)

A suspension of 2-amino-6-chloropurine (1) (25 g, 147 mmol) and potassium carbonate (41 g, 297 mmol) in DMF (500 ml) was stirred at room temperature for 1 h. Benzyl 2-bromoacetate (37 g, 25 ml, 162 mmol) was added in one portion and the reaction mixture was stirred for 3 h. After adding celite (10 g), the inorganic solid was removed by filtration through celite and the filtrate was evaporated to dryness in vacuo. The solid residue was suspended in DMF (125 ml) and cooled at 15°C, followed by filtration. The clear brown solution was evaporated in vacuo. The raw product was dissolved in hot ethanol (1.5 l) and kept over night at room temperature before filtration. This gave 34.6 g (74%) of the pure product 2. MS (+MALDI-TOF) 318.0 [M+H]^{+ 1}H NMR (d6-DMSO) δ 8.13 (1H, s, H-8); 7.41-7.35 (5H, m, Ph); 7.00 (2H, s, NH₂); 5.21 (2H, s, CH₂Ph); 5.08 (2H, s, CH₂CO).

### Benzyl (N²-(Isobutyryl)-2-amino-6-chloropurin-9-yl)acetate (3)

To a solution of Benzyl (2-amino-6-chloropurin-9-yl)acetate (2) (1.0 g, 3.14 mmol) in pyridine (10 ml) at 0°C was added isobutyrylchloride (0.4 g, 3.75 mmol). The mixture was stirred at 0°C for 30 min, followed by 3 h at room temperature. The mixture was evaporated to dryness in vacuo and the residue was dissolved in hot ethyl acetate. The product precipitate slowly when standing at 0°C. Yield= 800 mg (66%). MS (+MALDI-TOF) 389.4 [M+H]⁺; ¹H NMR (d6-DMSO) δ 10.82 (1H, s, NH); 8.52 (1H, s, H-8); 7.37 (5H, m, Ph); 5.23/5.22 (4H, 2xs, 2xCH₂); 2.82 (1H, m, CH-iBu); 1.10 (6H, d, CH₃-iBu).

### (N²-(Isobutyryl)-2-amino-6-chloropurin-9-yl)acetic acid (4)

Benzyl (*N*²-(isobutyryl)-2-amino-6-chloropurin-9-yl)acetate (600 mg, 1.5 mmol) was dissolved in a mixture of H₂O (4 ml) and THF (4 ml) and 2 M NaOH (6 ml) was added. The mixture was left with stirring for 1 h. After adjusting the pH at 2.5 with 2M NaHSO₄ the mixture was extracted with ethyl acetate (3x100 ml). The organic phase was dried over MgSO₄ and the filtrate concentrated in vacuo. The resulting yellow oil was purified by column chromatography using DCM/MeOH/triethylamine (90:10:1) as eluent. This gave 360 mg (78%) of the title compound 4. MS (+MALDI-TOF) 299.3 [M+H]⁺; ¹H NMR (d6-DMSO): δ10.76 (1H, s, NH); 8.42 (1H, s, H-8); 4.62 (2H, s, CH₂CO); 2.92 (6H, q, 3xCH₂-TEA); 2.80 (1H, m, CH-iBu); 1.10 (15H, m, 2xCH₃-iBu and 3xCH3-TEA).

### Methyl N-((N²-(Isobutyryl)-2-amino-6-chloropurin-9-yl)acetyl)-N-(2-Boc-aminoethyl)glycinate (5)

Methyl *N*-(*tert*-Butyloxycarbonylaminoethyl)glycinate (260 mg, 1.12 mmol) was dissolved in DMF (5 ml) and (*N*²*-*(Isobutyryl)-2-amino-6-chloropurin-9-yl)acetic acid (280 mg, 0.94 mmol), HOBt (160 mg, 1.2 mmol) and DCC (250 mg, 1.2 mmol) was added. After 2 h at room temperature the mixture was diluted with DCM and the solution was filtered and washed with 0.5 M NaHCO₃ and with 2 M NaHSO₄. After concentration in vacuo, the crude product was dissolved in ethanol/water and left over night at 4°C. The pure product was isolated upon filtration (320 mg, 0.62 mmol, 56%). MS (+MALDI-TOF) 512.0 [M+H]⁺; ¹H NMR (d6-DMSO): δ 10.82 (1H, s, NH); 8.38 (1H. s, H-8); 7.07-6.74 (1H, m, NH); 5.28/5.11 (2H, 2xs, rotamers CH₂CO); 4.48/4.10 (2H, 2xs. rotamers CH₂CO); 3.62 (3H, s, OCH₃); 3.61-3.19 (4H, m, 2xCH₂); 2.84 (1H, m, CH-iBu); 1.32 (9H, s, 3xCH₃-tBu); 1.09 (6H, d, 2xCH₃-iBu).

### N-((N²-(Isobutyryl)-2-amino-6-chloropurin-9-yl)acetyl)-N-(2-Boc-aminoethyl) glycine (6)

Methyl *N*-((*N*^{*2*}-(Isobutyryl)-2-amino-6-chloropurin-9-yl)acetyl)-*N*-(2-Boc-aminoethyl)glycinate (220 mg, 0.43 mmol) was dissolved in a mixture of THF (1 ml) and water (1 ml). After addition of 1 M LiOH (1 ml, 1 mmol) the mixture was left at room temperature for 2 h. THF was removed *in vacuo* and the pH was adjusted to 2.5 with 2 M NaHSO₄. The product was obtained by extraction with ethyl acetate and concentration of the organic phase. This gave 155 mg (72%) of the title compound. MS (+MALDI-TOF) 498.4 [M+H]^{+;} ¹H NMR (d6-DMSO): δ 10.82 (1H, s, NH); 8.39 (1H, s, H-8); 7.07-6.73 (1H, m, NH); 5.27/5.11 (2H, 2xs, rotamers CH₂CO); 4.35/4.02 (2H, 2xs, rotamers CH₂CO); 3.54-3.03 (4H, m, 2xCH₂); 2.84 (1H, m, CH-iBu); 1.32 (9H, s, 3xCH₃-tBu); 1.08 (6H, d, 2xCH₃-iBu).

### Example 2

### Nucleophilic displacement in 6-chloropurin monomer

The final monomer 6 was dissolved in ammonia in water (32%), methylamine in water (40%) and piperidine in DMF (20%), respectively. The reaction was followed by HPLC and with MALDI-TOF MS. The substitution of 6-chloro takes place within minutes as seen by HPLC and MS. The removal of the isobutyryl group is more slowly, fastest in methylamine (complete in 5 h at room temperature) and very slow in piperidine (not complete in 72 hours). For definitions see figure 5.
7: MS (+MALDI-TOF) 478.6 [M+H]⁺
8: MS (+MALDI-TOF) 492.5 [M+H]⁺
9: MS (+MALDI-TOF) 546.3 [M+H]⁺
10: MS (+MALDI-TOF) 407.9 [M+H]⁺
11: MS (+MALDI-TOF) 422.6 [M+H]⁺
12: MS (+MALDI-TOF) 476.1 [M+H]⁺

### Example 3

### Nucleophilic displacement of 6-chloropurin in PNA oligomer

The PNA Ac-TGT-ACG-TCG^{Cl}-CAA-CTA-Gly **(13)** was synthesized on an ABI 433A peptide synthesizer using Boc/Acyl monomers and *N*-((*N*^{*2*}-(Isobutyryl)-2-amino-6-chloropurin-9-yl)acetyl)-*N*-(2-Boc-aminoethyl)glycine **(6)**. The base-labile PAM linker to the MBHA-resin was introduced in the first cycle using Boc-Gly-PAM (double coupling). All cycles and reagents were standard as previously reported [Koch et al. J. Peptide Res. 49, 1997, 80-88]. The final PNA was split up in two tubes and treated in (A) ammonia in water (20 h at 60°C) and in (B) methylamine (5 h at room temperature).
14: MS (+MALDI-TOF) 4162.5 [M+H]⁺
15: MS (+MALDI-TOF) 4180.0 [M+H]⁺

## Claims

1. A method for the synthesis of compounds of the general formula I 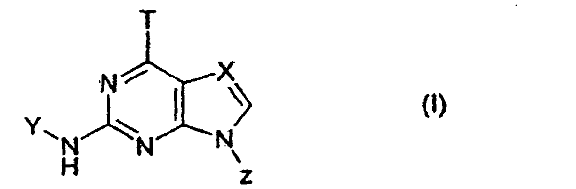 wherein
T is NV¹V², wherein V¹ and V² are linked together other than via the N shown in the definition by a chain of at least 5 atoms, or
T is NHV, wherein V is selected from the group consisting of hydrogen, alkyl and a protecting group,
X is CH or N,
Y is an electron withdrawing protecting group, preferably a group belonging to the group of unsubstituted or substituted acyl groups, and
Z is an alkyl group substituted with a derivative of a COOH group wherein the derivative is an ester, thioester or amide with an unprotected or protected peptide nucleic acid,
**characterized in that** a compound of general formula II 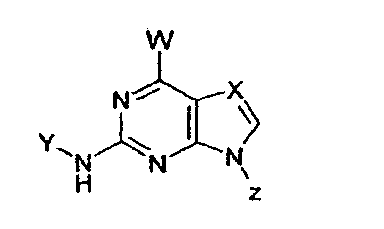 wherein the definitions of X, Y and Z are chosen from the possible definitions of X,Y and Z in formula I, and
W is an electron withdrawing group, preferably selected from the group consisting of NO₂, halogen, Tos and Mes,
is reacted with a compound of general formula III
T-H (III)
wherein T is defined as in formula I,
under non-alkaline conditions favouring the elimination of a compound of the formula WH.

## Patentansprüche

1. Verfahren zum Synthetisieren von Verbindungen der allgemeinen Formel I 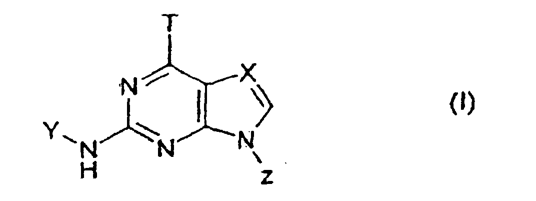 worin
T NV¹V² ist, worin V¹ und V² miteinander anders verknüpft sind als über das N, wie in der Definition gezeigt, durch eine Kette von mindestens 5 Atomen, oder
T NHV ist, worin V gewählt ist aus der Gruppe, bestehend aus Wasserstoff, Alkyl und einer Schutzgruppe,
X CH oder N ist,
Y eine Elektronen-entziehende Schutzgruppe ist, vorzugsweise eine Gruppe, die zur Gruppe der unsubstituierten oder substituierten Acylgruppen gehört, und
Z eine Alkylgruppe ist, die mit einem Derivat einer COOH-Gruppe substituiert ist, wobei das Derivat ein Ester, Thioester oder Amid mit einer ungeschützten oder geschützten Peptid-Nukleinsäure ist,
**dadurch gekennzeichnet, dass** eine Verbindung der allgemeinen Formel II 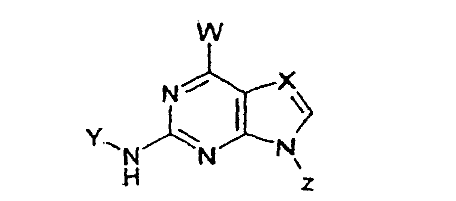 worin die Definitionen von X, Y und Z gewählt sind aus den möglichen Definitionen von X, Y und Z in Formel I, und
W eine Elektronen-entziehende Gruppe ist, vorzugsweise gewählt aus der Gruppe, bestehend aus NO₂, Halogen, Tos und Mes,
umgesetzt wird mit einer Verbindung der allgemeinen Formel III
T-H (III)
worin T wie in Formel I definiert ist,
unter nicht-alkalischen Bedingungen, die die Eliminierung einer Verbindung der Formel WH begünstigen.

## Revendications

1. Méthode de synthèse de composés de formule générale I 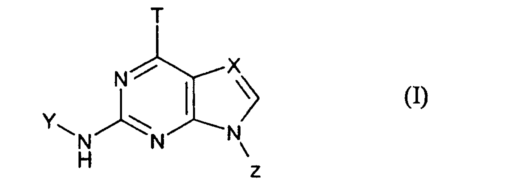 dans laquelle
T représente NV¹V², où V¹ et V² sont liés ensemble autrement que par l'intermédiaire du N représenté dans la définition par une chaîne d'au moins 5 atomes, ou
T représente NHV, où V est choisi parmi un groupe constitué d'un hydrogène, d'un alkyle et d'un groupement protecteur,
X représente CH ou N,
Y est un groupement protecteur électroattracteur, de préférence un groupement appartenant au groupe des groupements acyles substitués ou non substitués, et
Z est un groupement alkyle substitué par un dérivé de groupement COOH où le dérivé est un ester, un thioester ou un amide d'un acide nucléique peptidique protégé ou non protégé,
**caractérisée en ce qu'**un composé de formule générale II 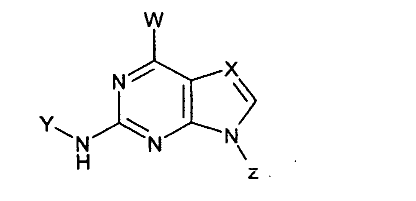 dans laquelle les définitions de X, Y et Z sont choisies parmi les définitions possibles de X, Y et Z dans la formule I, et
W est un groupement électroattracteur, choisi de préférence parmi le groupe constitué de NO₂, d'un halogène, de Tos et de Mes,
est mis à réagir avec un composé de formule générale III
T-H (III)
dans laquelle T est tel que défini dans la formule I,
dans des conditions non alcalines favorisant l'élimination d'un composé de formule WH.
